# FASCICULE DE BREVET EUROPEEN

(11) **EP 0 621 779 B1**
(45) Date de publication et mention de la délivrance du brevet: **05.10.2005**
(21) Numéro de dépôt: 93904072.1
(22) Date de dépôt: 12.01.1993
(51) Int. Cl.: A61K 31/405, A61K 31/198, C07D 209/28, A61P 27/02

(54) **LYSINATE D'INDOMETHACINE POUR L'UTILISATION THERAPEUTIQUE EN OPHTALMOLOGIE**
INDOMETHACIN-LYSINAT ZUR THERAPEUTISCHEN VERWENDUNG IN DER OPHTHALMOLOGIE
INDOMETHACIN LYSINATE FOR THERAPEUTIC USE IN OPHTHALMOLOGY

(30) Priorité: 13.01.1992 FR 9200381
(43) Date de publication de la demande: 02.11.1994
(73) Titulaire: JUNG, Jean, 67000 Strasbourg (FR); DONCOEUR, François, 78530 Buc (FR)
(72) Inventeur: Jung, Jean, 67000 Strasbourg (FR); Stoclet, Alain, 78400 Chatou (FR)
(74) Mandataire: Nuss, Laurent
(86) Numéro de dépôt international: PCT/FR1993/000023
(87) Numéro de publication internationale: WO 1993/013770

(56) Documents cités:
- EP-A- 0 324 402
- EP-A- 0 424 028
- DE-A- 2 508 895
- DE-A- 3 531 279
- FR-A- 2 217 003
- FR-A- 2 569 690
- RES.VET.SCI. vol. 37, no. 1, Juillet 1984, pages 26 - 29 A.REGNIER ET AL. 'EFFECT OF LYSINE-ACETYLSALICYLATE AND PHENYLBUTAZONE PREMEDICATION ON THE PROTEIN CONTENT OF SECONDARY AQUEOUS HUMOUR IN THE DOG'
- ANN.ALLERGY vol. 67, no. 1, Juillet 1991, pages 60 - 62 G.PATRIARCA ET AL. 'NASAL PROVOCATION TEST WITH LYSINE ACETYLSALICYLATE IN ASPIRIN-SENSITIVE PATIENTS'
- OFTALMOL.ZH. no. 1, 1986, pages 55 - 57 D.S-KROL' ET AL. 'THE USAGE OF ACETYLSALICYLATE-LYSINE IN CORRECTION OF HEMODYNAMIC DISTURBANCES IN EYE CONTUSION IN EXPERIMENT' SUMMARY
- ANN.ALLERGY vol. 67, no. 6, Décembre 1991, pages 588 - 592 G.PATRIARCA ET AL. 'INTRANASAL TREATMENT WITH LYSINE ACETYLSALICYLATE IN PATIENTS WITH NASAL POLYPOSIS'
- CHEMICAL ABSTRACTS, vol. 104, 1986, Columbus, Ohio, US; abstract no. 102160k,
- CHEMICAL ABSTRACTS, vol. 105, 1986, Columbus, Ohio, US; abstract no. 107897m,
- PATENT ABSTRACTS OF JAPAN vol. 11, no. 349 (C-456)(2796) 13 Novembre 1987
- PATENT ABSTRACTS OF JAPAN vol. 11, no. 115 (C-415)(2562) 10 Avril 1987
- PATENT ABSTRACTS OF JAPAN vol. 7, no. 46 (C-153)(1191) 23 Février 1983
- PATENT ABSTRACTS OF JAPAN vol. 7, no. 83 (C-160)(1228) 7 Avril 1983

## Description

La présente invention concerne le domaine des médicaments et a pour objet un procédé de fabrication d'un médicament destiné à une utilisation thérapeutique en ophtalmologie.

Actuellement l'indométacine est utilisé en ophtalmologie sous forme de collyre.

D'une part, il s'agit d'une suspension à un pour cent d'indométacine, en l'occurrence l'INDOCID® fabriqué par les laboratoires MERCK-SHARP et DOHME CHIBRET. Mais ce collyre présente différents inconvénients : irritation des tissus superficiels de l'oeil, difficile maîtrise de la taille des cristaux, les plus petits ayant tendance à disparaître en augmentant la taille des plus gros, présence de polymorphisme, entraînant une assez mauvaise biodisponibilité qui peut varier en fonction du temps de conservation du médicament.

D'autre part, il existe des composés arylacétiques, en particulier de l'indométacine sous forme de collyre dont la dissolution de l'indométacine lyophilisé est obtenue par action d'un milieu tampon à base de borate-acide borique. Il s'agit de l'INDOCOLLYRE® fabriqué par les Laboratoires CHAUVIN. Mais la forme soluble de l'indométacine n'a pas été isolée ni identifiée. Or, il est préférable pour un médicament d'avoir un principe actif défini, isolé, caractérisable par des méthodes physico-chimiques permettant une étude de stabilité de la matière première selon les réglementations de nombreux pays dans le cadre du contrôle de qualité. En outre, la préparation industrielle de ce collyre est très complexe et nécessite l'emploi de nombreux excipients.

La formule des deux collyres INDOCID® et INDOCOLLYRE® est la suivante :
- INDOCID® Collyre : suspension à 1 % de cristaux d'indométacine (flacon de 3 ml).

| Formule pour 100 ml | |
|---|---|
| indométacine micronisée | 1,00 g |
| lécithine | (0,02 g) |
| sulfite monosodique | 0,02 g |
| chlorure de sodium | (0,60 g) |
| polysorbate 80 | (0,0375 g) |
| hydroxyéthylcellulose QP 100 MH | (0,15 g) |
| chlorure de benzalkonium | 0,02 g |
| alcool benzylique | 0,25 g |
| alcool phényléthylique | 0,25 g |
| solution de sorbitol à 70 pour cent | (1,00 g) |
| édétate de sodium | 0,05 g |
| eau distillée q.s.p. | 100,0 ml |

Les valeurs indiquées entre parenthèses sont indicatives et ne figurent pas dans l'ouvrage VIDAL® 1991 édité par Editions du Vidal®, 11 rue Quentin-Bauchart 75384 Paris Cedex 08.
Le pH du collyre est compris entre 4,0 et 6,0.
- INDOCOLLYRE® : solution à 0,1 % d'indométacine sous forme de flacon de lyophilisat et de flacon de solvant de 5 ml.

| Lyophilisat (par flacon) : | |
|---|---|
| indométacine | 5,0 mg |
| dextran | (20,0 mg) |
| acide borique | (28,1 mg) |
| borax | (5,1 mg) |

| Solvant (pour 100 ml) : | |
|---|---|
| acide borique | (1,995 g) |
| borax | (0,149 g) |
| parahydroxybenzoate de méthyle | 0,050 g |
| P.E.G. 400 | (24,850 g) |
| édétate disodique | (0,050 g) |
| eau purifiée q.s.p. | 100,00 ml |

| Formule du collyre reconstitué (pour 100 ml) : | |
|---|---|
| indométacine | 0,1000 g |
| borax | (0,2510 g) |
| acide borique | (2,5569 g) |
| dextran | (0,4000 g) |
| polyéthylène glycol 400 | (24,8500 g) |
| parahydroxybenzoate de méthyle | 0,0500 g |
| édétate disodique | (0,0500 g) |
| eau purifiée q.s.p. | 100 ml |

Les valeurs indiquées entre parenthèses sont indicatives et ne figurent pas dans l'ouvrage VIDAL® 1991.
Le pH du collyre reconstitué est compris entre 6,5 et 7,1.

On connaît enfin du document CHEMICAL ABSTRACTS, vol. 98, 1983, Columbus, Ohio, US ; abstract n° 78165 & JP-A-57,197,211, l'utilisation de l'indométacine avec la 1-arginine en tant que collyre. Mais la préparation du produit fait intervenir du mannitol, ce qui nécessite une lyophilisation. Par ailleurs, le mannitol présent ne permet pas une stérilisation à une température de + 170°C. Enfin, la préparation du collyre sans du mannitol est impossible.

Le document DE-A-2508895 décrit un procédé de préparation de sels d'acides phényl-alcanoïques avec des acides aminés basiques dans lequel la salification est effectuée dans un milieu principalement aqueux avec un excès de solvant organique (acétone, diéthyléther, isopropanol...) susceptible d'irriter l'oeil. Les composés divulgués dans ce document étant principalement destinés à une application par injection ou par voie parentérale, ils ne disposent pas d'une biodisponibilité et innocuité suffisantes pour leur administration directe à la surface de l'oeil.

La présente invention a pour but de pallier ces inconvénients.

Elle a, en effet, pour objet, un procédé de fabrication d'un médicament destiné à une utilisation thérapeutique en ophtalmologie du lysinate d'indométacine selon la revendication 1.

Le document Patent Abstracts of Japan, 7, No. 46 (C-153) (1191) 23 Février 1983, & JP-A-57197211 décrit l'utilisation d'une composition pharmaceutique comprenant de l'indométhacine et de l'arginine en ophtalmologie (abrégé). Le document Patent Abstracts of Japan, 7, No. 46 (C-153) (1191) 23 Février 1983, & JP-A-57200361 décrit l'utilisation d'une composition pharmaceutique comprenant de l'indométhacine, de l'arginine et du béta-cyclodextrine en ophtalmologie (abrégé). Le document Patent Abstracts of Japan, 7, No. 83 (C-160) (1228) 7 Avril 1983, & JP-A-5810517 décrit l'utilisation d'une composition pharmaceutique comprenant du ketoprophène et de la lysine en ophtalmologie (abrégé). Le document Patent Abstracts of Japan, 11, No. 349 (C-4566)(2796) 13 Novembre 1987, & JP-A-62123119 décrit l'utilisation d'une composition pharmaceutique comprenant du lysinate d'acide acetylsalicylique dans les opérations ophtalmologiques. L'inhibition du myosis pendant une opération ophtalmologique est améliorée en utilisant ladite composition pharmaceutique en combinaison avec un collyre comprenant de l'indométhacine (abrégé). Le document Chemical Abstracts, vol. 104, 1986, abrégé no. 102160k, & Atarashii Ganka, vol. 2, no. 9, 1985, pages 1290-1292, N. Sato et al. "Effects of aspirin or dexamethasone on prostaglandin E in the lens of uveitic rabbit eyes" décrit l'utilisation de l'aspirine DL-lysine pour l'inhibition de la production du prostaglandine E dans les lentilles des lapins (abrégé), Le document Chemical Abstracts, vol. 105, 1986, abrégé no. 107897m, & Atarashii Ganka, vol. 3, no. 3, 1986, pages 377-379, & Y. Kawabata et al. "Ocular penetration of topically applied aspirin DL-lysine" décrit l'utilisation de l'aspirine DL-lysine en ophtalmologie (abrégé), Le document par Regnier et al., "Effect of lysine-acetylsalicylate and phenylbutazone premedication on the protein content of secondary aqueous humour in the dog"; Res. Vet. Sci. 1984, vol. 37, no. 1, pages 26-29 décrit l'utilisation de l'acetylsalicylate de la lysine comme agent anti-inflammatoire en ophtalmologie (abrégé). Le document Patent Abstracts of Japan, 11, No. 115 (C-415)(2562) 10 Avril 1987, & JP-A-61260020 décrit l'utilisation de l'acetylsalicylate de la lysine en ophtalmologie (abrégé).

Le contrôle de qualité du sel utilisé doit être réalisé selon les directives de la FDA (Food and Drug Administration) et des Communautés Européennes comme par exemple les Directives de la Commission CEE N° 75/318/CEE et modifications, N° 91/507/CEE.

Conformément à une caractéristique de l'invention, le composé utilisé sous forme de sel peut, par exemple, être une combinaison entre l'indométacine et la lysine, composé amino-acide, c'est-à-dire le lysinate d'indométacine.

Le composé sous forme de sel conforme à l'invention entre dans des compositions médicamenteuses du type collyre associés à différents ions, contenant, par exemple, le NaH₂PO₄, le Na ₂HPO₄ et le NaCl afin d'obtenir une solution aqueuse isotonique et présentant un pH voisin de la neutralité.

Dans la préparation des collyres le pH joue un rôle important car il existe plusieurs valeurs optimales de pH qui sont généralement différentes :
- pH optimal pour la conservation chimique du principe actif
- pH optimal d'activité thérapeutique, pH optimal de tolérance. Le pH du collyre est ajusté à celui des larmes au moyen de solutions tampons.

Dans le cas présent, un tampon phosphate de pH = 6,85 peut par exemple être utilisé. Sa formule est la suivante :

| | |
|---|---|
| NaH₂PO₄,2H₂O | 10,2 g |
| Na₂HPO₄ anhydre | 11,1 g |
| eau distillée q.s.p. | 1000,0 g |

Un exemple de formule de collyre peut être :

| | |
|---|---|
| lysinate d'indométacine | 0,200 g |
| solution tampon (pH = 6,85) | 50 ml |
| sel isotoniant (NaCl) | 0,432 g |
| eau distillée q.s.p. | 100 ml |

Des mélanges de solution aqueuse et de suspension peuvent être envisagés.

Le composé sous forme de sel conforme à l'invention peut également entrer dans des compositions médicamenteuses du type gel associant un mélange gélifiant pouvant par exemple être du carbopol, du mercurothiolate de sodium, du mannitol, de l'hydroxyde de sodium et de l'eau, afin d'obtenir un gel au pH voisin de la neutralité. La formule quantitative peut par exemple être la suivante :

| | |
|---|---|
| lysinate d'indométacine | 0,180 g |
| carbopol | 0,320 g |
| mannitol | 4,900 g |
| hydroxyde de sodium M | 3,000 g environ |
| mercurothiolate de sodium | 0,004 g |
| eau distillée q.s.p. | 100 ml |

Le composé sous forme de sel utilisé peut également entrer dans des compositions médicamenteuses du type émulsion associant une phase aqueuse contenant le sel à une phase lipidique transformées en une émulsion.

Le procédé conforme à la présente invention consiste à faire réagir la fonction carboxylique avec la fonction amine en milieu hydro-éthanolique en dehors de tout autre solvant organique conduisant à un produit final dépourvu de tout autre solvant résiduel que l'éthanol, des traces d'éthanol pouvant être éventuellement présentes.

### Mode opératoire:

204 g de L-(+)-lysine sont dissous dans un mélange de 30 1 d'éthanol 95° et de 5 1 d'eau. 500 g d'indométacine sont rajoutés par petites portions sous agitation. Il y a formation d'un précipité qui est filtré. Le précipité est lavé avec de l'éthanol puis séché à l'étuve à 100°C. On obtient une poudre blanche. Masse théorique obtenue : 704 g ; rendement moyen : 86 %.

Cette méthode de préparation peut être transposée ; les proportions peuvent être un multiple par exemple de 10, de 100, de 1000.
Formation du sel entre l'indométacine et la L(+)lysine

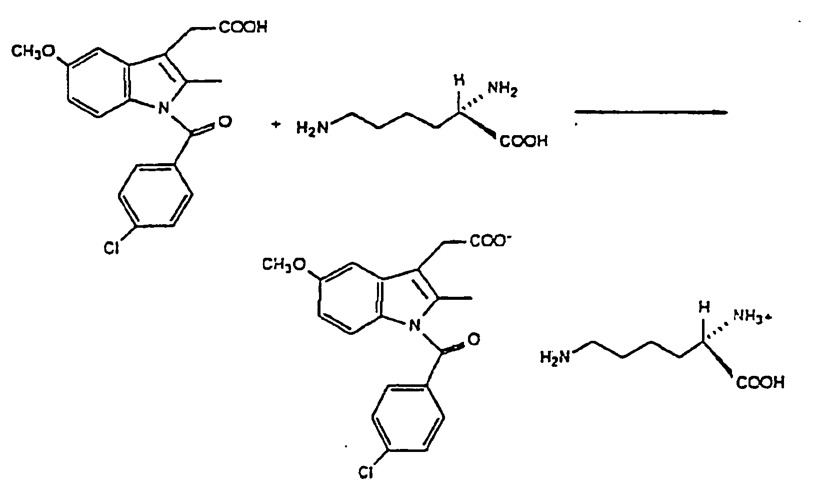

Le composé sous forme de sel obtenu est une poudre aux propriétés physico-chimiques définies, stables dans le temps dans des conditions classiques de conservation.

Caractères physico-chimiques du lysinate d'indométacine obtenu : Poudre cristalline blanche à jaune, inodore ou sensiblement inodore, soluble dans l'eau et le méthanol, pratiquement insoluble dans l'acétone, le chloroforme et le dichlorométhane, totalement insoluble dans l'acétonitrile, l'éther et l'éthanol. Les données des études spectrales (IR, UV, RNM) sont conformes à la structure définie.

Le composé sous forme de sel isolé à l'état pur est stérilisable, soit à l'état pur, soit en solution aqueuse, par exemple par autoclavage ou par filtration sur membrane.

Le lysinate d'indométacine destiné à la préparation d'un collyre doit subir une étape de stérilisation. Plusieurs méthodes peuvent être utilisées :
1. Stérilisation par la chaleur sèche ou humide : cette méthode est facile à mettre en oeuvre, elle nécessite comme matériel un four du type Poupinel, ou un autoclave à vapeur.
2. Stérilisation par irradiation : cette méthode est plus lourde à mettre en oeuvre. En effet, elle nécessite des installations spécifiques. La dose d'irradiation doit être déterminée afin d'obtenir un produit stérile, produit non dégradé. La mise en oeuvre de ce procédé de stérilisation est plus complexe qu'une stérilisation à l'autoclave.
3. Stérilisation par un gaz tel l'oxyde d'éthylène ou le formaldéhyde : cette méthode est assez lourde à mettre en oeuvre. De plus, il y a risque d'apparition d'effets toxiques au cours de l'utilisation du produit si la concentration en gaz résiduel dans le produit est trop élevée.
4. Filtration sur membrane : cette méthode est utilisée pour les solutions. Des précautions doivent être prises afin de s'assurer du maintien des propriétés du filtre durant son utilisation (étanchéité et stérilité du filtre).

Le lysinate d'indométacine sous forme de poudre a été stérilisé suivant la première méthode, la chaleur sèche, à une température supérieure à 170°C sans être dégradé.

Dans un premier temps, la stabilité du lysinate d'indométacine en fonction de la température a été prouvée et les conditions de température et de durée utilisées pour la stérilisation ont été définis. Dans un deuxième temps, une étude microbiologique a permis de vérifier l'efficacité de la méthode.

La stérilisation de la solution aqueuse du lysinate d'indométacine a été réalisée par filtration sur membrane ; elle donne un produit stérile.

La solution aqueuse isotonique ou la suspension ou le gel ou l'émulsion conforme à l'invention peut être utilisé en thérapeutique dans le cas, par exemple, des manifestations inflammatoires pouvant être liées aux interventions chirurgicales de la cataracte et du segment antérieur de l'oeil, pour la prévention de l'oedème maculaire cystoïide de l'aphaque survenant après extraction chirurgicale du cristallin.

Le passage oculaire est généralement très faible ; 5 % au maximum de la dose instillée sont retrouvés dans l'humeur aqueuse de l'oeil.

Les biodisponibilités oculaires des deux formes pharmaceutiques sont équivalentes : le passage obtenu avec la solution à 0,142 % d'indométacine (du lysinate d'indométacine) est similaire à celui obtenu avec la suspension à 1 % (INDOCID®). Cette dernière constatation permet d'affirmer que le passage des collyres à travers les différentes barrières oculaires s'effectue bien par la fraction soluble et non par endocytose de microparticules.

Il faut noter également qu'au temps de prélèvement de 2 h les concentrations d'indométacine mesurées dans l'oeil traité par la solution de lysinate d'indométacine à 0,142 % d'indométacine sont supérieures à celles mesurées dans l'oeil traité par la suspension à 1 % d'indométacine ; cela peut être dû à une élimination plus rapide de la forme suspension par le fluide lacrymal en raison de l'irritation oculaire provoquée par la taille des particules.

La cinétique d'absorption de l'indométacine est grossièrement la suivante : 10 minutes après l'instillation, la concentration de l'indométacine dans l'humeur aqueuse est pratiquement nulle. Elle augmente ensuite régulièrement pour atteindre sa valeur maximale environ 30 minutes après l'instillation (3 à 5 % de la dose instillée) et diminue finalement.

## Revendications

1. Utilisation du lysinate d'indométhacine pour la fabrication d'un médicament stérile destiné à une utilisation thérapeutique en ophtalmologie, ledit lysinate d'indométhacine dérivant d'une salification de la fonction carboxylique par le groupement aminé d'un amino-acide, dans laquelle ledit lysinate d'indométhacine pénètre dans les tissus et les humeurs de l'oeil, sous la forme d'une solution aqueuse isotonique et de pH neutre ou voisin de la neutralité, ou sous la forme d'une suspension, d'un gel ou d'une émulsion contenant d'autres ions, ledit lysinate d'indométhacine étant présent dans ladite solution, suspension, gel ou émulsion en tant que seul principe actif sous la forme d'un sel isolé à l'état pur et dépourvu de tout solvant résiduel sauf, le cas échéant, de traces d'éthanol.

2. Utilisation selon la revendication 1 pour le traitement des manifestations inflammatoires pouvant être liées aux interventions chirurgicales de la cataracte et du segment antérieur de l'oeil, pour la prévention de l'oedème maculaire cystoïide de l'aphaque survenant après extraction chirurgicale du cristallin.

3. Utilisation selon la revendication 1 ou 2, **caractérisée en ce que** dans la solution aqueuse isotonique, les ions sont des tampons contenant, du NaH₂PO₄, du Na₂HPO₄ ou du NaCl.

4. Utilisation selon l'une quelconque des revendications 1 à 3, **caractérisé en ce que** dans le gel, le mélange gélifiant est du carbopol, du mercurothiolate de sodium, du mannitol, de l'hydroxyde de sodium et de l'eau.

5. Utilisation selon l'une quelconque des revendications 1 à 4, **caractérisé en ce que** le lysinate d'indométhacine est obtenu en faisant réagir la fonction carboxylique avec la fonction amine en milieu hydro-éthanolique en dehors de tout autre solvant organique conduisant à un produit final dépourvu de tout autre solvant résiduel que l'éthanol.

6. Utilisation selon la revendication 5, **caractérisé en ce que** le sel isolé à l'état pur est stérilisé soit à l'état pur, soit en solution aqueuse, par exemple par autoclavage ou par filtration sur membrane.

## Patentansprüche

1. Verwendung von Indomethacin-Lysinat zur Herstellung einer sterilen pharmazeutischen Zusammensetzung oder Arzneimittel zum therapeutischen Einsatz in der Ophthalmologie, wobei das Indomethacin-Lysinat gebildet wird durch Salzbildung der Carboxylfunktion mit der Aminogruppe einer Aminosäure, wobei das Indomethacin-Lysinat in Form einer isotonischen wässrigen Lösung mit neutralem oder nahezu neutralem pH-Wert oder in Form einer Suspension, eines Gels oder einer Emulsion, die andere Ionen enthält, in die Gewebe und die Flüssigkeiten bzw. das Kammerwasser des Auges eindringt, wobei das Indomethacin-Lysinat in der Lösung, der Suspension, dem Gel oder der Emulsion als einziges Wirkprinzip in Form eines isolierten Salzes im Reinzustand und ohne jeden Lösungsmittelrückstand gegebenenfalls abgesehen von Spuren von Ethanol vorliegt.

2. Verwendung gemäß Anspruch 1 zur Behandlung von Entzündungserscheinungen, die im Zusammenhang mit einer Staroperation oder einer Operation am vorderen Augenabschnitt aufgetreten sein können, zur Verhinderung eines zystoiden Makulaödems bei nach chirurgischer Linsenextraktion auftretender Aphakie.

3. Verwendung nach Anspruch 1 oder 2, **dadurch gekennzeichnet, dass** die Ionen in der isotonischen wässrigen Lösung Puffer sind, die NaH₂PO₄, Na₂HPO₄ oder NaCl enthalten.

4. Verwendung nach einem der Ansprüche 1 bis 3, **dadurch gekennzeichnet, dass** die gelbildende Mischung in dem Gel Carbopol, Natriumquecksilberthiolat, Mannitol, Natriumhydroxid und Wasser ist.

5. Verwendung nach einem der Ansprüche 1 bis 4, **dadurch gekennzeichnet, dass** das Indomethacin-Lysinat erhalten wird, indem die Carboxylfunktion mit der Aminfunktion in einem wässrig-ethanolischen Medium ohne jedes andere organische Lösungsmittel reagieren gelassen wird, was zu einem Endprodukt führt, das keinerlei Lösungsmittelrückstände außer Ethanol enthält.

6. Verwendung nach Anspruch 5, **dadurch gekennzeichnet, dass** das isolierte reine Salz entweder im Reinzustand oder in wässriger Lösung sterilisiert wird, und zwar beispielsweise durch Autoklavieren oder durch Membranfiltration.

## Claims

1. Use of indomethacin lysinate for the preparation of a sterile pharmaceutical composition intended for therapeutic use in ophthalmology, said indomethacin lysinate deriving from salification of the carboxylic function by the amino group of an amino acid, wherein said indomethacin lysinate penetrates the tissues and the humours of the eye, in the form of an aqueous isotonic solution and having a neutral or approximately neutral pH, or in the form of a suspension, a gel or an emulsion containing other ions, said indomethacin lysinate being present in said solution, suspension, gel or emulsion as the single active ingredient in the form of a salt isolated in its pure state and without any residual solvent except, if appropriate, traces of ethanol.

2. Use according to claim 1 for the treatment of inflammatory manifestations that may be associated with surgical procedures carried out on cataracts and the anterior segment of the eye, for the prevention of aphakic cystoid macular oedema occurring after surgical extraction of the crystalline lens.

3. Use according to claim 1 or 2, **characterised in that**, in the aqueous isotonic solution, the ions are buffers containing NaH₂PO₄, Na₂HPO₄ or NaCl.

4. Use according to any one of claims 1 to 3, **characterised in that**, in the gel, the gelling mixture is carboxypolymethylene, sodium mercurothiolate, mannitol, sodium hydroxide and water.

5. Use according to any one of claims 1 to 4, **characterised in that** the indomethacin lysinate is obtained by reacting the carboxylic function with the amino function in a hydroethanol medium, in the absence of any other organic solvent leading to a final product without any residual solvent other than ethanol.

6. Use according to claim 5, **characterised in that** the salt isolated in its pure state is sterilised either in its pure state or in an aqueous solution, for example by autoclaving or by membrane filtration.
